(19) Europäisches Patentamt
European Patent Office
Office européen des brevets

(11) Veröffentlichungsnummer: **0 348 507 A1**

(12) # EUROPÄISCHE PATENTANMELDUNG

veröffentlicht nach Art. 158 Abs. 3 EPÜ

(21) Anmeldenummer: **88902621.7**

(22) Anmeldetag: **23.12.87**

Daten der zugrundeliegenden internationalen Anmeldung:
(86) Internationale Anmeldenummer: **PCT/SU 87/00151**
(87) Internationale Veröffentlichungsnummer: **WO 89/05660 (29.06.89 89/14)**

(51) Int. Cl.⁴: **A 61 K 47/00,** A 61 K 9/12 // (A61K47/00, 31:11, 31:10)

(43) Veröffentlichungstag der Anmeldung: **03.01.90 Patentblatt 90/1**

(84) Benannte Vertragsstaaten: **BE CH DE FR GB IT LI**

(71) Anmelder: **POLTAVSKY MEDITSINSKY STOMATOLOGICHESKY INSTITUT, ul. Shevchenko, 23,, Poltava 314000 (SU)**

(72) Erfinder: **KRAVCHENKO, Vladimir Grigorievich, ul. Zygina, 4-60, Poltava, 314014 (SU)**

(74) Vertreter: **von Füner, Alexander, Dr. et al, Patentanwälte v. Füner, Ebbinghaus, Finck Mariahilfplatz 2 & 3, D-8000 München 90 (DE)**

(54) **ARZNEIMITTELZUBEREITUNG FÜR DIE INDIVIDUELLE PROPHYLAXE VENERISCHER KRANKHEITEN UND ZUR HEILUNG VON UROGENITALER TRICHOMONIASE.**

(57) Das Arzneimittel zur individuellen Prophylaxe venerischer Erkrankungen und Behandlung der urogenitalen Trichomoniasis enthält eine effektive Menge einer aktiven Komponente, die ein synergisches Gemisch aus para-Nitro-α-chlorzimtaldehyd der Formel

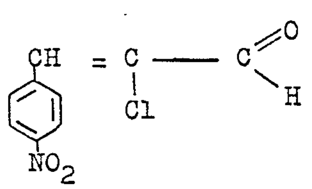

und Dimethylsulfoxid darstellt, und ein pharmazeutisches Verdünnungsmittel. Es wird vorgeschlagen, als pharmazeutisches Verdünnungsmittel Polyäthylenglykol mit der Molekularmasse 400 zu verwenden.

EP 0 348 507 A1

0 348 507

# ARZNEIMITTEL ZUR INDIVIDUELLEN PROPHYLAXE VENERISCHER ERKRANKUNGEN UND ZUR BEHANDLUNG DER UROGENITALEN TRICHOMONIASIS

## Gebiet der Technik

Die vorliegende Erfindung bezieht sich auf das Gebiet der Medizin und betrifft insbesondere ein Arzneimittel zur individuellen Prophylaxe venerischer Erkrankungen und zur Behandlung der urogenitalen Trichomoniasis.

## Zugrundeliegender Stand der Technik

Zur Zeit sind in der Medizin Mittel zur individuellen Prophylaxe venerischer Erkrankungen weist bekannt, solche wie Silberpräparate, Kaliumpermanganat und Quecksilberpräparate, beispielsweise Ätzsublimatlösung, Kalomelsalbe, Salbe "Dublosan". Zwecks Prophylaxe von Syphilis und Gonorrhoe wurde das Arzneimittel "Oxyviridol" vorgeschlagen, das aus 0,075 Masse-% Quecksilberoxyzyanid und grüner Seife in Glyzerin (50 Masse-% in Glyzerin) besteht /M.Kh. Bergolts "Vrachebny retsepturny spravochnik" (Ärztliches Rezepturhandbuch), 1952, Medgiz, Moskau/. Dieses Arzneimittel weist jedoch keine genügende bakterizide Wirksamkeit gegenüber Erregern von Syphilis und Gonorrhoe auf, übt eine bedeutende Reizwirkung auf die Schleimhaut der Urethra aus, ruft Abschuppung und Lysis des Epithelüberzugs hervor und ist in der klinischen Anwendung nicht ungefahrlich. Die aufgezählten Arzneimittel finden keine breite praktische Verwendung wegen deren ungenügender Zuverlässigkeit, und die Quecksilberpräparate - wegen fehlender Garantie der Unschädlichkeit für den menschlichen Organismus.

In den letzten Jahren wurde als Arzneimittel zur Prophylaxe venerischer Krankheiten 0,05 - 0,1%ige Chlorhexidinlösung (Hibitan) angeboten. Es besitzt eine genügende bakterizide Wirksamkeit bei Experimenten in vitro und bei Versuchen an Tieren (Vestnik dermatologii i venerologii, Nr. 6, 1978; Moskau, N.M. Ovchinnikov u.a. "Zur Frage über die persönliche Prophylaxe venerischer Krankheiten", S. 49). Jedoch wird bei seiner klinischer Anwendung eine reizende und austrocknende Wirkung auf die Haut und Schleimhaut der menschlichen Geschlechtsorgane beobachtet. Die Herstellung

von Hibitan unter Betriebsverhältnissen ist kompliziert und verlangt einen großen Arbeitsaufwand.

Zur äußerlichen Behandlung der urogenitalen Trichomoniasis werden normale Borsäure, Höllenstein, Zinksulfat, Kupfersulfat, Lävomycetin, Osarsol u.a. angewandt. Diese Arzneimittel sind nicht effektiv genug und finden keine breite Verwendung.

Zur Zeit sind Imidazolpräparate, beispielsweise Tinidazol, Metronidazol, Trichopol die effektivsten Mittel zur Behandlung dieser Erkrankung. Jedoch wird bei ihrer klinischer Anwendung immer wachsende Tricnomonasresistenz gegen deren Wirkung beobachtet, was die Rezidivierung des spezifischen Prozesses (bis 8,4% der Fälle) und die Entwicklung der posttrichomonaden Erscheinungen bedingt. Diese Arzneimittel können Nebenwirkungen auslösen und sind bei Störungen des Blutbildungssystems und aktiven Erkrankungen des Zentralnervensystems kontraindiziert.

Offenbarung der Erfindung

Der vorliegenden Erfindung liegt die Aufgabe zugrunde, ein Arzneimittel zur individuellen Prophylaxe venerischer Erkrankungen und zur Behandlung der Trichonomiasis in bequemer Arzneiform durch Auswahl als aktive Komponente solcher chemischer Verbindungen zu entwickeln, die eine Effektivität des Präparats ohne Nebenerscheinungen bei dessen Anwendung gewährleisten.

Die Aufgabe wird dadurch gelöst, daß ein Arzneimittel zur individuellen Prophylaxe venerischer Erkrankungen und zur Behandlung der urogenitalen Tricnomoniasis, enthaltend eine aktive Komponente und ein pharmazeutisches Verdünnungsmittel, vorgeschlagen wird, das erfindungsgemäß eine effektive Menge der aktiven Komponente enthält, welche ein synergisches Gemisch aus para-Nitro-α-chlorzimtaldehyd der Formel

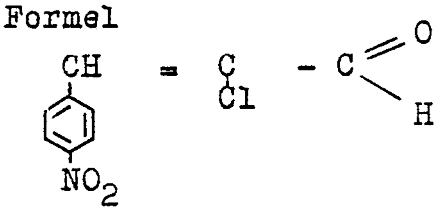

und Dimethylsulfoxid darstellt.

Die chemischen Verbindungen des Gemisches, das als aktive Komponente vorgeschlagen wird, besitzen verschiedene Wirkungsmechanismen, die insgesamt einen potenzierenden Synergismus erzeugen, der zu einer vollen Destruktion des Protoplasmas und der Wand der Erreger von Syphilis, Gonorrhoe und Trichomoniasis führt und einen schnell ausgeprägten bakteriziden und protistoziden Effekt dem Arzneimittel verleiht. Unter dem Einfluß des Präparats geht der Zerfall der Erreger innerhalb der ersten 3 Minuten vor sich. Das Präparat besitzt eine hohe Effektivität sowohl in der Prophylaxe venerischer Erkrankungen, als auch für die Behandlung der orogenitalen Trichomoniasis.

Es ist zweckmäßig, als pharmazeutisches Verdünnungsmittel Polyäthylenglykol mit der Molekularmasse 400 auszunutzen. Es weist keine Toxizität auf und ist eine osmotisch aktive Substanz, die dazu beiträgt, daß die aktive Komponente ihre höchste biologische Aktivität zeigt.

Das Arzneimittel mit einem solchen Verdünnungsmittel besitzt keine lokale Reizwirkung auf die Haut und Schleimhaut, keine allgemein toxische und allergisierende Wirkung.

Zur klinischen Anwendung wird das Arzneimittel in flüssiger Form bei folgendem Komponentenverhaltnis (Masse-%) empfohlen:

| | |
|---|---|
| para-Nitro-$\alpha$-chlorzimtaldehyd der obengenannten Formel | 0,3 |
| Dimethylsulfoxid | 5,0 |
| Polyäthylenglykol mit der Molekularmasse 400 | bis 100. |

Ein solches Arzneimittel besitzt die höchste Zuverlässigkeit der prophylaktischen Wirkung und der therapeutischen, gegen Trichomonas wirkenden Aktivität. Das Arzneimittel in flüssiger Arzneiform ermöglicht den besten Kontakt desselben mit auf die Haut und Schleimhaut durchgedrungenen Erregern.

Es empfiehlt sich, zur individuellen Prophylaxe venerischer Erkrankungen und Behandlung der urogenitalen Trichomoniasis insbesondere bei Frauen das Arzneimittel in der Aerosolverpackung unter Druck in flüssiger Form zu

verwenden, welches die Komponenten bei folgendem Verhältnis (Masse-%) enthält:

| | |
|---|---|
| para-Nitro-α- chlorzimtaldehyd der genannten Formel | 0,3 |
| Dimethylsulfoxid | 5,0 |
| Polyäthylenglykol mit der Molekularmasse 400 | 20-30 |
| oberflächenaktive Substanz | 3,0-6,0 |
| Wasser | bis 100 |

sowie Propellent in einer Menge von 3 bis 10 % von der Gesamtmasse der Komponenten.

Eine solche Arzneimittelform bietet die Möglichkeit, notwendigenfalls nicht nur den antivenerischen Effekt sicherzustellen, sondern auch die Selbständigkeit therapeutischer Prozeduren den Frauen zu gewährleisten, die einer Behandlung der urogenitalen Trichomoniasis bedürfen.

Beste Ausführungsform der Erfindung

Das erfindungsgemäße Arzneimittel stellt eine dicke durchsichtige Flüssigkeit von einer schwach gelben bis zur dunkelgelben Farbe mit einem schwachen spezifischen Geruch dar, die bei der Aufbewahrung an lichtgeschützter Stelle stabil ist.

Das Arzneimittel kann in hermetisch verschlossenen Flaschen mit 5 bis 10 ml Inhalt oder in der Aerosolverpakkung hergestellt werden.

Das erfindungsgemäße Arzneimittel zur Prophylaxe venerischer Erkrankungen und zur Behandlung der Trichomoniasis bereitet man durch Vermischen effektiver Mengen von para-Nitro- α-chlorzimtaldehyd der Formel

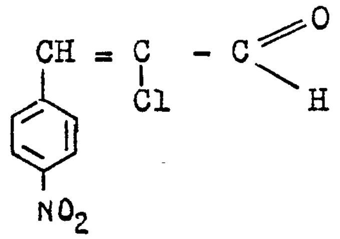

und von Dimethylsulfoxid bis zur Bildung eines synergischen Gemisches in Form von Lösung und durch dessen Verdünnen bis zu einer vorgegebenen Konzentration mit einem pharmazeutischen Verdünnungsmittel - Polyäthylenglykol mit der

Molekularmasse 400 - zu. Dieses Verdünnungsmittel wurde wegen seiner sehr guten Kompatibilität mit den aktiven Komponenten und wegen seiner geringen Toxizität gewählt. Für diesen Zweck können auch andere von den angewandten pharmazeutischen Verdünnungsmitteln, beispielsweise Glyzerin, Propylenglykol, 1,2-Propandiol, verwendet werden.

Bei der Herstellung des erfindungsgemäßen Arzneimittels als Druckaerosol in flüssiger Form gibt man der vorstehend beschriebenen Lösung eine oberflächenaktive Substanz (3 bis 6 Masse-%), Wasser bis 100 Masse-% zu und sättigt die erhaltene Lösung in der Aerosolverpackung mit einem Propellenten in einer Menge von 3 bis 10 % von der Masse der Komponenten. Die Mengen dieser Substanzen werden ausgehend von der Herstellung eines bei der Anwendung sich schnell zerstörenden Schraums gewählt.

Es wurden vorklinische Prüfungen des erfindungsgemäßen Arzneimittels durchgeführt, welche die Untersuchung seiner bakterizid-protistozider Wirksamkeit in Versuchen in vitro und mit experimenteller Syphilis bei Kaninchen, die Untersuchung seiner Unschädlichkeit, darunter die Untersuchung der akuten und chronischen Toxizität, der lokalen Reizwirkung, der Allergie erzeugenden Wirkung, des Einflusses auf das Immunsystem sowie einige Seiten der Pharmakokinetik einschließen.

Das Prüfarzneimittel wurde in Konzentrationen von 0,075 %, 0,15 % und 0,3 % untersucht. Die Konzentration wurde nach der Menge des im Arzneimittel enthaltenen para-Nitro-$\alpha$-chlorzimtaldehyds der genannten Formel beim ständigen mengenmäßigen Gehalt an Dimethylsulfoxid (5%) und äquivalent veränderlichen Gehalt an Polyäthylenglykol mit einer Molekularmasse von 400 bestimmt. Die Untersuchung der Unschädlichkeit des Arzneimittels wurde bei einer Konzentration von 0,3 % durchgeführt.

Zur Untersuchung der gonococcoziden Eigenschaften des Arzneimittels wird die reine Gonokokkuskultur von den Gonorrhoekranken isoliert und auf eine große Fläche des Nährmediums ("Matratzen") geimpft. Die 24 Stunden alte Kultur wird von den Matratzen mit steriler isotonischer Nat-

riumchloridlösung abgewaschen, und aus der Abwaschflüssigkeit wird eine Suspension zubereitet, die I Mlrd Mikrobenkörper (nach dem "Trübungsstandard") enthält. Die erhaltene Suspension wird in sterile Reagenzgläser zu 0,5 ml abgefüllt und zu jedem Reagenzglas werden je 0,5 ml Prüfarzneimittel der doppelten Konzentration zugegeben, um nach dem Vermischen der Ingredienzien innerhalb von 5 min die erforderliche (zu untersuchende) Konzentration zu erhalten. Dieses Gemisch wurde innerhalb von 10 bis 15 min bei 1500 bis 3000 U/min zentrifugiert (das Abwaschen der Gonokokken vom Arzneimittel). Die Flüssigkeit über dem Niederschlag wurde entfernt, und zum Niederschlag wurde wiederum I ml isotonische Natriumchloridlösung (bis zum Erreichen des anfänglichen Volumens) hinzugefügt und nochmals zentrifugiert (wiederholtes Abwaschen). Die Flüssigkeit über dem Niederschlag wurde entfernt und der Niederschlag wurde auf den schrägen Agar des Nährmediums geimpft (für jeden Stamm der Gonokokkuskultur wurden je 10 Reagenzglaser mit dem Nährmedium ausgenutzt). Die Reagenzgläser mit dem Nährmedium wurden in einem Thermostat bei einer Temperatur von 37 °C in der Atmosphare mit einem erhöhten Gehalt an Kohlensäure untergebracht. Das Wachstum der Gonokokkuskultur wurde jede 24 Stunden im Laufe von 4 Tagen bestimmt.

Als Kontrolle diente das Gemisch von gleichen Volumina der Suspension der Gonokokkuskultur und der isotonischen Natriumchloridlösung (zu 0,5 ml), das in Reagenzgläser zweimal ebenso wie in den Versuchsreagenzgläsern abgefüllt, zentrifugiert und, der Niederschlag auf ein der Zusammensetzung nach identisches Nährmedium geimpft wurde.

Als Nährmedium wurde der Fleisch-Pepton-Agar aus dem Kaninchenfleisch unter Zugabe von Kaseinhydrolysat, Hefeautolysat und Rindviehblutserum ohne Konservierungsmittel sowie ein lyophiles Medium ausgenutzt.

Die Wirkung des Arzneimittels auf Treponema pallidum und vaginale Trichomonas wurde in den Versuchen folgenderweise untersucht.

Treponema pallidum wurde aus 8 bis IO Tage alten spezifischen Hodenentzündungen der Kaninchen und von an ansteckenden Syphilisformen leidenden Kranken erhalten. Es wurden auch die Gewebekulturen der Nichols-Stamme und der Stämme Nr. 8 des Zentralen dermatovenerologischen Forschungsinstituts (ZKVI) untersucht. Die Kulturen der vaginalen Trichomonas wurden von den an urogenitaler Trichomoniasis isoliert Kranken und auf einem künstlichen Nährmedium von Johnson-Trussell gezüchtet.

Die Versuchsmethodik bestand in der Vermischung gleicher Volumina der Suspension von Treponema pallidum (oder der Trichomonaskultur) und des Prüfarzneimittels die in doppelter Konzentration genommen werden, weil sich die Konzentration beim Vermischen um das Zweifache vermindert. Aus dem Gemisch werden native Präparate zubereitet und im Dunkelfeld (für Treponema pallidum und Trichomonas) oder Phasenkontrastfeld (für vaginale Trichomonas) eines Mikroskops mikroskopiert. Die Wirkung des Prüfpräparats wurde nach dem Grad und der Geschwindigkeit dessen Einflusses auf die Beweglichkeit und Morphologie der Erreger beurteilt. Diese Wirkung wurde als positiv eingeschätzt, wenn die volle Bewegungslosigkeit der Erreger sofort eintrat, d.h. im Moment der Zuvereitung des Präparats, oder in den nächsten 3 bis 5 min für Treponema und 5 bis 10 min für Trichomonas. Für positiver hielt man die Einwirkung des Prüfpräparats auf die Morphologie von Treponema und Trichomonas, wenn die Krankheitserreger in derselben Periode ihre Form verloren (Treponema pallidum wurde dünn, die Schnörkel glichen sich aus, es traten aufgeschwollene Individuen, Bruchstücke auf, oder es wurde völlig aufgelöst; Vaginaltrichomonas verlor Geißelfäden, zerfiel oder wurde aufgelöst.

Eine andere Variante der Untersuchung des treponemoziden Effekts des Prüfarzneimittels bestand im aufeinanderfolgenden Auftragen eines Tropfens der Suspension von Treponema pallidum und eines Tropfens des zu untersuchenden Arzneimittels auf den Objektträger, das Gemisch wurde mit dem scharfen Ende der Pasteurpipette vermischt, mit einem Deckglas bedeckt und sofort im Dunkelfeld des Mikroskops untersucht. Diese Untersuchung gibt die Möglichkeit, die Einwirkung des Prüfarzneimittels auf Treponema schon in den nächsten 20 bis 30 sec nach dem Kontakt der Suspension und des Arzneimittels an den Tag zu legen. Als Kontrolle für diese Versuche diente ein Gemisch gleicher Volumina der Suspension von Treponema pallidum (von 20-30 bis 80 Exemplare im Sehfeld) oder von Vaginaltrichomonas und isotonischer Natriumchloridlösung. Die mikroskopischen Präparate wurden im Dunkelfeld des Mikroskops "Biolam" und mit Hilfe des Mikroskops der Firma "Opton" unter Ausnutzung von Dunkelfeld- und Phasenkontrastkondensoren und Objektiven (25 x 0,45; 40 x 0,60) untersucht.

Zur Untersuchung der prophylaktischen Wirkung des Arzneimittels in den Versuchen mit experimenteller Syphilis wurden geschlechtsreife, wohlgenährte 2,5 bis 4 kg schwere männliche Kaninchen von verschiedener Rasse und Haarfarbe (Chinchilla, weißes und graues silberschimmerndes Riesenkaninchen) ausgenutzt.

Vor den Versuchen befanden sich alle Kaninchen unter Beobachtung während mindestens eines Monats, um die spontane Kaninchenspirochätose auszuschließen. Sie alle wurden vor den Versuchen unter Anwendung eines Komplexes serologischer Reaktionen auf Syphilis untersucht.

Der prophylaktische Effekt des Prüfarzneimittels wurde in den Versuchen mit experimenteller Kaninchensyphilis in zwei parallelen Versuchsvarianten untersucht.

Die erste Variante sah die Untersuchung des treponemoziden Effektes des Präparats bei der intrakutanen Einführung des Gemisches der Suspension von Treponema pallidum und dem Arzneimittel vor. Zu diesem Zweck wurden den nach dem üblichen Verfahren fixierten Versuchskaninchen in beide Hodensackhälften je 0,75 ml Gemisch intrakutan eingeführt, das aus gleichen Volumina der Suspension von Treponema pallidum und der doppelten Konzentration des zu untersuchenden Arzneimittels bestand (weil sich dessen Konzentration beim Vermischen mit der Suspension dementsprechend um das Zweifache verminderte).

Der einen Tiergruppe wurde das obengenannte Gemisch sofort nach 10 min langem Kontakt der Ingredienzien und deren Vermischen mit einem sterilen Glasstäbchen eingeführt. Der anderen Kaninchengruppe wurde das Gemisch nach dessen Zentrifugieren nach 10 min langem Kontakt des Volumens der sterilen isotonischen Natriumchloridlösung eingeführt (Abwaschen vom Arzneimittel).

Diese Untersuchungen wurden durchgeführt, um die Möglichkeit der Inaktivierung (Fällung) des Arzneimittels im Proteinmedium des Organismus der Tiere auszuschließen und auf diese Weise dessen bakteriziden Effekt zu vernichten. Durch die Einführung der Treponemasuspension nach dem Zentrifugieren wurde die Erzielung eines genügenden treponemoziden Effekts des Arzneimittels nach dessen 10 min langer Einwirkung auf Treponema pallidum vorgesehen. Den Kontrollkaninchen wurde das Gemisch aus gleichen Volumina der Suspension von Treponema pallidum und isotonischer Natriumchloridlösung in die Hodensackgegend intrakutan eingeführt.

Die zweite Variante- die Hauptvariante der Versuche - bestand in der Durchführung der Versuche, bei welchen die Bedingungen der Syphilisinfizierung der Kaninchen in bestimmtem Maße der Syphilisinfizierung der Menschen unter natürlichen Bedingungen nahekamen. Zu diesem Zweck wurden die Tiere mit der Suspension von Treponema pallidum in isotonischer Natriumchloridlösung durch deren Einreibung in die skarifizierte Hodensackhaut infiziert. Vorläufig wird die Hodensackhaut mit einem mit Alkohol, nachher mit Äther befeuchteten Wattetampon gesäubert. Danach wird die Hodensackhaut mit den Fingern der linken Hand maximal ausgedehnt, und mit der rechten Hand wird die Haut von beiden Hodensackhälften nacheinander mit feinkörnigem Schmirgelpapier skarifiziert. Die Skarifikation von 1 bis 1,5 $cm^2$ Fläche muß eine solche sein, daß eine rauhe Oberfläche bis zum stellenweisen Auftreten kleiner Punkte der Gewebeflüssigkeit entsteht. Das Auftreten von Blutungen ist zu vermeiden. Darauf wird die frisch zubereitete Suspension des Syphiliserregers, die 20 bis 25 Treponema pallidum im Sehfeld des Mikroskops enthalt, in einer Menge von 0,1 ml auf skarifizierte Gegenden der Hodensackhaut aufgetragen und mit einem sterilen Glasspatel während 1 bis 1,5 min eingerieben. Dann wird die Hodensackhaut mit zwei Peanschen Klemmen in einem Abstand von mindestens 0,5 cm von der Stelle des Auftragens des ansteckenden Materials ergriffen und die Klemmen werden miteinander gekreuzt, was zur Bildung einer Hautfalte führt, die Treponema gegen Austrocknung schützt (um die besten Bedingungen für die Ansteckung zu schaffen - nach S.T. Pavlov). Die Klemmen bleiben etwa 1 bis 1,5 Stunden gekreuzt dann werden sie weggenommen.

Die Versuchskaninchen wurden der Desinfektionsbehandlung mit dem zu testenden Arzneimittel in verschiedenen Zeitspannen nach der Infizierung (in 15 min, in 1, 2 und 3 Stunden) ausgesetzt. Die Behandlung bestand im Auftragen und einem sorgfältigen Einreiben mit einem sterilen Glasspatel von 0,1 ml des Testarzneimittels in jede infizierte Gegend der Hodensackhaut innerhalb von 1 bis 1,5 min. Die Kontrollkaninchen wurden der Desinfektionsbehandlung nicht unterworfen.

Die Versuchstiere wurden im Laufe von 4 bis 12 Monaten unter allwöchentlicher klinischer und allmonatlicher serologischer Untersuchung unter Anwendung eines Komplexes serologischer Reaktionen auf Syphilis beobachtet: wassermannsche Reaktion mit treponemalen und lipoiden Kardiolipin-Antigenen; Flockungsreaktionen nach Kahn und Sachs-Witebsky, Reaktion der Immunofluoreszenz (FIF-10 und RIF-200) und Reaktionen der Mikropräzipitation (RMP) beobachtet.

Die Zuverlässigkeit der prophylaktischen Geschütztheit der Versuchskaninchen vor Syphilis wurde nach dem Ausbleiben bei ihnen klinischer Äußerungen der Erkrankung, nach negativen serologischen Reaktionen sowie nach negativen Ergebnissen der Passageimpfung, der Kniekehlen-Lyphknoten von den Versuchstieren zu den Kaninchen-Indikatoren in zwei aufeinanderfolgenden Passagen eingeschätzt. Als endgültiger Beweis galten positive Ergebnisse der Reinokulation des homologen Stammes von Treponema pallidum den Versuchs- und Passagekaninchen.

In allen Versuchen wurde die frisch hergestellte Suspension von Treponema pallidum des Nicholsschen Stammes ausgenutzt.

Unten werden die Ergebnisse der experimentellen Untersuchung der spezifischen Wirksamkeit des Arzneimittels angeführt.

Bakterizide Wirkung des Arzneimittels auf Neissersche Gonokokken. In den Versuchen mit zwölf von 7 kranken Frauen und 5 Männern isolierten Erregerstämmen wurde festgestellt, daß das Arzneimittel die Lebenstätigkeit der Gonokokkenkulturen vollständig unterdrückt. In keinem Versuchsreagenzglas wurde das Wachstum der Gonokokkenkultur nachgewiesen, während in allen Kontrollreagenzgläsern deren intensives Wachstum zu verzeichnen war.

Treponemozide Wirksamkeit des Arzneimittels. Durch Versuche wurde festgestellt, daß unter dem Einfluß des Arzneimittels eine fast augenblickliche Bewegungslosigkeit von Treponema pallidum und gleichzeitig die Zerstörung der Syphiliserreger bis zu deren voller Lysis während der ersten 3 min nach dem Kontakt mit dem Arzneimittel eintritt. Schon in der ersten Minute sind einzelne Fetzen zerstörter Treponema, lange, unbewegliche,dünne "Fäden", "Schatten" (weil sich die Schnörkel ausgleichen) und in den meisten Präparaten - die Anhäufung einer amorphen Proteinsubstanz in Form von kleinen Teilchen aus lysierten Erregern zu sehen. Einer ähnlichen zerstörenden Einwirkung unter dem Einfluß des Arzneimittels werden Treponema pallidum sowohl des Nicholsschen Stammes als auch des Stammes Nr. 8 ZKVI ausgesetzt. Es wurden auch keine Unterschiede im Grad der treponemoziden Wirksamkeit des Arzneimittels in bezug auf Erreger festgestellt, die unmittelbar von den an ansteckenden Syphilisformen kranken isoliert wurden. Der treponemozide Effekt wurde auch in Versuchen mit experimenteller Kaninchensyphilis bestätigt. Insgesamt wurden in den Versuchen 120 männliche Kaninchen ausgenutzt. Die Verhütung der Tiefe vor der

Syphilisinfektion wurde in 100 % der Fälle nach der prophylaktischen Bearbeitung der Infizierungsstelle mit dem Arzneimittel innerhalb von 15 min bis 3 Stunden nach dem Auftragen des ansteckenden Materials auf die skarifizierte Haut des Kaninchenhodensacks erreicht. Gleichzeitig wurden positive Ergebnisse der Syphilisprophylaxe bei der intrakutanen Infizierung der Kaninchen mit dem Gemisch aus der Suspension von Treponema pallidum in der isotonischen Natriumchloridlösung und dem Arzneimittel erzielt, die in gleichen Volumenverhältnissen genommen wurden.

Die Zuverlässigkeit der prophylaktischen Geschütztheit der Versuchskaninchen vor Syphilis wurde sowohl durch das Ausbleiben bei ihnen klinischer Äußerungen der Erkrankung und durch negative serologische Reaktionen auf Syphilis (die Tiere standen unter Beobachtung von 4 bis 12 Monaten), als auch durch die negativen Ergebnisse der Passageimpfung der Kniekehlen-Lymphknoten von den Versuchskaninchen zu den Kaninchen-Indikatoren in zwei aufeinanderfolgenden Passagen bestätigt: von den Versuchstieren zu den Kaninchen-Indikatoren der ersten Passage und von ihnen - den Tieren-Indikatoren der zweiten Passage. Als Beweis galten auch positive Ergebnisse der Reinokulation mit dem homologen Stamm von Treponema pallidum sowohl bei Versuchs- als auch bei Passagekaninchen.

Die Verhütung der Versuchstiere vor Syphilis bei der intrakutanen Infizierung zeugt davon, daß eine 10 min lange Exposition des Kontakts von Arzneimittel und Treponema pallidum vollkommen ausreichend ist, um die Lebenstätigkeit der Syphiliserreger vollständig zu unterdrücken. Andererseits deutet es auch auf die Stabilität des Arzneimittels im Proteinmedium hin, und die Gewebe des Kaninchenorganismus stellen gerade solch ein Medium dar.

Wirkung des Arzneimittels auf Trichomonas vaginalis. In Versuchen im Reagenzglas mit 16 Stammen der vaginalen Trichomonas, die von den an urogenitaler Trichomoniasis kranken Frauen (13) und Männern (3) isoliert wurden, wurde eine hohe protistozide Wirksamkeit des Arzneimittels

festgestellt (der Untersuchung wurden 256 mikroskopische Präparate unterzogen). Unter Einwirkung des Arzneimittels werden die vaginalen Trichomonas schon in der ersten Minute bewegungslos und verlieren Geißeln. Innerhalb der nächsten 2 bis 3 min geht der Zerfall der Protozoen vor sich und nur in einzelnen mikroskopischen Präparaten wurden vereinzelte bewegungslose nicht zerfallene Trichomonas ohne Geißeln beobachtet.

Um die Unschadlichkeit des Arzneimittels zu untersuchen, wurden von uns akute und chronischen Toxizität, lokale Reizeigenschaften auf die Haut und Schleimhaut, Allergie erzeugende und immunogene Eigenschaften bestimmt. Untersucht wurde auch die Pharmakokinetik des Arzneimittels bei der dermatischen Anwendung und beim Auftragen auf die Schleimhäute.

Die Bestimmung der akuten Toxizität des Arzneimittels wurde an 4 Tierarten durchgeführt. In 4 Versuchsserien wurden 150 weiße Mäuse von 18,5 g durchschnittlicher Körpermasse, 18 weiße Ratten (165 g), 18 Kaninchen (3000 g) und 12 Meerschweinchen (400 g) ausgenutzt. Die Einzeldosis des zu testenden Arzneimittels wurde unter Berücksichtigung dessen zukünftiger Anwendung für praktische Ziele berechnet (0,07 ml pro 1 kg Körpermasse des bedingten Menschen von 70 kg durchschnittlicher Körpermasse).

Unter Berücksichtigung der Spezifik der praktischen Anwendung des Arzneimittels (für die Einsalbung in die Haupt und Einführung in die Urethra) wurde die Cidipol--Toxizität außer der intraperitonealen Einführung (weiße Ratten, weiße Mäuse) auch bei der dermatischen und intrakavitären Einführung - in die Scheide bei Weibchen und ins Rektum bei Männchen (weiße Ratten, Meerschweinchen und Kaninchen) untersucht. Visuelle Beobachtung des Zustandes der Versuchstiere wurde sofort nach der Anwendung des Testarzneimittels vorgenommen und dauerte 14 Tage.

Die Untersuchung der chronischen Cidipol-Toxizität wurde an zwei Labortierarten durchgeführt: an 24 weißen Ratten von 170 bis 200 g Körpermasse und 24 Kaninchen der Chinchilla-Rasse von 2,5 bis 3,0 kg Körpermasse. Das Arzneimittel wurde täglich im Laufe eines Monat angewendet. Die Einzeldosis des Arzneimittels wurde ebenso wie bei der Untersuchung der akuten Toxizität mit Rücksicht auf dessen praktischer Anwendung zwecks Prophylaxe berechnet.

Einer der Versuchsgruppen der Tiere (Kaninchen und Ratten - je 6 Stück)wurde täglich im Laufe eines Monats die oben erwähnte therapeutische Arzneimitteldosis eingeführt. Der anderen Versuchsgruppe beider Tierarten (je 6 Stück) wurde täglich während der ersten zwei Wochen die subtoxische Dosis des Arzneimittels eingeführt, die um das 10-fache die therapeutische Dosis - 0,7 ml/kg übertrifft, und von der 3. Woche an bis zum Ende des Monats setzte man die Einführung des Arzneimittels den Kaninchen in einer Dosis von 2,1 ml/kg, den Ratten - in einer Dosis von 3,5 ml/kg fort. Die letzten Dosen übertrafen die therapeutische Dosis um das 30 bzw. 50-fache. Eine Gruppe der Kaninchen und Ratten (je 6 Stück) diente als Kontrollgruppe, in welcher statt des Arzneimittels isotonische Natriumchloridlösung angewendet wurde. Der Einführungsweg des Arzneimittels den Versuchstieren war den Bedingungen der Medikation beim Menschen maximal angenähert. Den weiblichen Tieren wurde das Arzneimittel in die Scheide und ins Rektum (1/3 der Dosis) eingeführt sowie auf die vom Fell befreite Haut der inneren Schenkel- und Bauchoberfläche aufgetragen und innerhalb von 1 min eingerieben (2/3 der Dosis); den Männchen wurde das Arzneimittel ins Rektum eingeführt (1/3 der Dosis) und in die Haut des Hodensacks, des Bauches und der inneren Schenkeloberfläche eingerieben (2/3 der Dosis).

Während der ganzen Beobachtungsperiode wurden bei den Tieren das Allgemeinbefinden, das Verhalten fixiert, es wurden die Dynamik der Körpermasse und die rektale Temperatur bestimmt. Es wurde der Einfluß des Arzneimittels auf die Funktionen des Zentralnervensystems (ZNS), des kardiovaskulären und des Atemsystems, der Leber, Nieren, Endokrindrüsen, des Bluts untersucht, einschließlich der Durchführung biochemischer Untersuchungen der Tätigkeit

des Herzens, der Lungen, Nieren, Leber, Entdokrindrüsen. Nach Beendigung der monatlichen Behandlungskur wurden die Tiere umgebracht und es wurden die histomorphologischen Untersuchungen von Gehirn und Rückenmark, Herzen, Lungen, Gefäßen, Leber, Nieren, Pankreas, Geschlechtsdrüsen, Blut, Blutbildungsorganen (Milz, Lymphknoten) durchgeführt. Außerdem wurden die Haut- und Schleimhautgegenden der Scheide und des Rektums untersucht, wohin das Arzneimittel eingeführt wurde, und die Gewichtskoeffizienten der inneren Organe bestimmt. Die Körpermasse und die rektale Temperatur wurden einmal pro Woche gemessen. Die übrigen physiologischen Indexe wurden vor und nach dem Versuch registriert. Die biochemischen Untersuchungen wurden dreimal durchgeführt - vor dem Versuch, nach zwei Wochen und nach einem Monat, d.h. nach der Beendigung des Versuchs.

Als Indexe der chronischen Toxizität bei Tieren dienten: der äußere Zustand und das Verhalten; die Dynamik der Temperatur und der Körpermasse; physiologische Tests (die Methode des "offenen Feldes", die Probe mit dem Hexenalschlaf, EKG); klinische Harnanalysen; integrale morphologische Daten des peripheren Buts; biochemische Blutindexe (die Bestimmung des gesamten, direkt und indirekt reagierenden Bilirubins, Kreatinins, Harnstoffes, Reststickstoffes, der Aminotransferasen, der Aktivität der alkalischen Phosphatase, der Aktivität der Blutserumcholinesterase, der $\alpha$-Amylase, des Gesamtproteins, Thymol trübungsprobe, die Bestimmung des C-reaktiven Proteins); die Bestimmung von Mikroelementen: Kalium, Calzium, Chloriden; pathomorphologische Untersuchungen der inneren Organe der Tiere.

Die funktionelle Aktivität des Nervensystems wurde nach der Methode des "offenen Feldes" untersucht, indem die motorische Aktivität und die Verhaltensreaktionen der Ratten innerhalb von 3 min nach der Zahl der Durchläufen, Wäschen und Stellungen registriert wurden. Außerdem wurde der funktionelle Zustand des ZNS nach der Dauer der latenten Periode bei der Durchführung der Probe mit dem Hexenalschlaf eingeschätzt, der durch die intraperitonale Einführung der 0,7%igen Hexenallösung in einer Dosis von 70,0 mg/kg hervorgerufen wurde.

Die elektrische Herzaktivität wurde mit Hilfe des Elektrokardiographen ELKAR 0,87 in der II. Standardableitung bei einer Bandtransportgeschwindigkeit von 50 mm/sec registriert. Bei der Bearbeitung der Elektrokardiogramme wurde die Herzfrequenz, die Länge des vollen Herzzyklus, der Intervalle CRS, ORST und die Voltage der Zacken O, R, S,T bestimmt.

Die wasserausscheidende und Konzentrationsfunktion der Nieren wurde nach dem Volumen der 24 Stunden langen Diurese nach der Flüssigkeitsbelastung (5 ml isotonische Natriumchloridlösung pro 100 g Tiergewicht) und nach der spezifischen Harnmasse eingeschätzt. Die stickstoffausscheidende Funktion der Nieren wurde nach der Konzentration des Reststickstoffs, des Harnstoffs sowie nach dem Kreatiningehalt im Blutserum eingeschätzt.

Die Blutprobe bei Ratten wurde aus dem abgehackten Schwanzende, bei Kaninchen - aus der peripheren Ohrvene genommen. Hämoglobin wurde photometrisch nach der unifizierten Hämoglobin-Zyanid-Methode unter Ausnutzung eines Standardsatzes der Reagenzien bestimmt. Erythrozyten wurden photometrisch am FEK-56 M bestimmt: vorläufig wurde nach der photometrischen Methode und durch die Bestimmung der absoluten Erythrozytenzahl in der Gorjaev--Kammer festgestellt, daß 0,1 Einh. der optischen Dichte 1,217 Mill. Erythrozyten in 0,01 ml Blut entspricht. Die Blutsenkungsgeschwindigkeit, die Gesamtzahl der Leukozyten und die Leukozytenformel wurden nach routinemäßigen Methoden bestimmt. Der Gehalt an Bilirubin, Kreatinin, Harnstoff, Aminotransferasen, am gesamten und C-reaktiven Blutprotein wurde nach unifizierten Methoden unter Ausnutzung der Standardsätze der Standardsätze der Reagenzien bestimmt.

Die Proben der inneren Organe für die Pathomorphologie wurden bei dekapitierten (Ratten) oder mit Hilfe der Luftembolie getöteten Tieren (Kaninchen) entnommen. Die Probemuster wurden im 10%igen neutralen Formalin fixiert

und mit Hämotoxylin-Eosin gefärbt. Gleichzeitig wurden die Masse und die Massekoeffizienten der inneren Organe bestimmt.

Die lokale Reizwirkung des Arzneimittels auf die Haut und Schleimhaut wurde in drei Versuchsserien untersucht. In der ersten Serie wurden 35 Meerschweinchen und 30 Kaninchen der Chinchilla-Rasse ausgenutzt, denen das Arzneimittel in die vom Fell befreiten Hautgegenden eingerieben und in den Konjunktivalraum beider Augen (ein- - oder zweimalig pro Tage im Laufe von 10 Tagen) eingeführt wurde.

In der zweiten Versuchsserie (8 Kaninchen) wurde die lokale Reizwirkung des Arzneimittels auf die Augenschleimhaut der Tiere untersucht, namlich in einer Konzentration, die um das 10fache die für die praktischen Ziele empfohlene Konzentration übersteigt. Wie in der ersten Serie wurde das Arzneimittel in beide Augen innerhalb von 10 Tagen ein- oder zweimal am Tag in einem Intervall von 2 bis 3 Stunden eingetröpfelt.

In der dritten Versuchsserie (bei der Untersuchung der chronischen Toxizität) wurde die lokale Reizwirkung des Arzneimittels unter den zu dessen praktischer Anwendung maximal angenäherten Bedingungen untersucht. Den Kaninchen (12 Tiere) und weißen Ratten (12 Stück) wurde das Arzneimittel täglich auf die Haut aufgetragen und in die Scheide (den Weibchen) und ins Rektum (den Männchen) während eines Monats eingeführt.

Außer der täglichen Beobachtung des Verhaltens der Tiere und der Reaktion seitens der Haupt und der Schleimhäute wurden nach Beendigung der Versuche die Haut- und Schleimhautabschnitte, die der Arzneimitteleinwirkung unterzogen wurden, für histologische Untersuchungen entnommen.

Die Untersuchung des allergisierenden Einflusses des Arzneimittels wurde an Meerschweinchen durchgeführt, weil diese Tiere das optimalste Objekt für die künstliche Sensibilisierung darstellen (Ado A,D., 1970). Den Versuchstieren wurde das Arzneimittel täglich im Laufe von 6

Tagen auf die Haut aufgetragen oder mit der Spritze in die Höhlen eingeführt (in die Scheide den Weibchen und ins Rektum den Männchen). Die Dosen des Arzneimittels waren therapeutisch (0,07 ml/kg) oder überstiegen die therapeutische Dosis um das 10- und 15fache (0,7 und 1,1 ml/kg). Die auslösende Dosis, die der summarischen sensibilisierenden Dosis gleich ist, wurde nach 21 bis 23 Tagen eingeführt. In den Versuchen wurden 24 Meerschweinchen ausgenutzt (vier Gruppen zu je 6 Stück, eine davon - Kontrollgruppe). Der Grad der allergisierenden Wirkung wurde nach visuellen Merkmalen (Hyperämie, Mikrobläschenbildung, Haut- und Schleimhautinfiltration, unadäquates Verhalten der Tiere, Gangstörung, Tremor u.a.) sowie nach den Ergebnissen der immunologischen Labortests eingeschätzt: Reaktion der Leukozytenagglomeration, Leukopenieprobe, Thrombozytopenieindex und Veränderung der Zahl von eosinophilen und basophilen Leukozyten im peripheren Blut.

In Übereinstimmung mit der oben erwähnten Methodik wurde die Erhöhung des Prozentsatzes der Leukozytenagglomeration um mehr als 30 %, die Verminderung der Leukozytenzahl um 1000 in 1 $mm^3$ Blut, die Erhöhung des Thrombozytopenieindexes um mehr als 22 % ebenso wie Eosinophilie--Basophilie für die Äußerung der Sensibilisierung gegenüber dem Arzneimittel gehalten.

Der Einfluß des Arzneimittels und seiner Grundlage auf das immune System wurde an 24 Kaninchen bei der Untersuchung der chronischen Toxizität untersucht (4 Gruppen zu je 6 Tiere, eine davon - Kontrollgruppe). Immunologische Untersuchungen wurden zweimal durchgeführt: zuerst wurde das Ausgangsniveau der Indexe bestimmt und nachher wurde nach einem Monat, d.h. nach Beendigung der Arzneimitteleinführung in der therapeutischen und subtoxischen Dosis und der Arzneimittelgrundlage in der subtoxischen Dosis die zweite Untersuchung unternommen. Es wurden folgende Tests angewendet, die die T- und B-Glieder der Immunität sowie unspezifische Schutzfaktoren kennzeichnen: die quantitative Bestimmung des Niveaus der Immunoglobuline der Klasse G, A und M im Blutserum (nach der Methode der radialen Immunodiffusion nach Mancini, 1965), die Veränderung der Zahl der Lymphozyten- und Monozytenzellen, Titer des Blutserumkomplements (kolorimetrische Methode), Titer der heterophilen Agglutinine (in der Reaktion nach Paul-Munnel), die Menge des C-reaktiven Proteins und des Gesamtblutserumproteins, histologische Untersuchungen der Milz und der Kniekehlenlymphknoten.

Das das Arzneimittel für die lokale Anwendung (auf die Haut und Schleimhaut) vorgesehen wird, scheint es wichtig zu sein, dessen mögliche Resorption von der Haut und Schleimhaut sowie die Möglichkeiten seines Metabolismus im Organismus zu bestimmen. Seine Bestimmung im Blut wurde nach hochempfindlichen Methoden der Gas-Flüssigkeit-Chromatographie durchgeführt, die gestatten, dieses in Konzentrationen von $10^{-6}$ bis $10^{-9}$ g/ml zu bestimmen.

Die statistische Bearbeitung der Zahlenangaben wurde am Elektronenrechner EC-1045 vorgenommen.

Die Ergebnisse der oben durchgeführten Versuche sind wie folgt.

Unter Bedingungen des akuten Versuchs bei der intraperitonealen Einführung des Arzneimittels weißen Mäusen wurden die minimalen toxischen Außerungen in einer Dosis von 8,32 ml/kg, die maximalen - in einer Dosis von 20,0 ml/kg Körpermasse der Tiere nachgewiesen. Die erhaltenen Angaben bezüglich der Untersuchung der akuten Toxizität wurden statistisch nach der Methode von B.M. Shtabsky u.a. (1988) bearbeitet. $LD_{50}$ für weiße Mäuse bei der intraperitonealen Einführung des Arzneimittels beträgt 9,81 ml/kg (9,67 bis 9,94), was laut der Toxizitätsklassifikation nach Sidorov K.K. gestattet, das Arzneimittel zu verhältnismäßig unschädlichen zu rechnen, was der niedrigsten Toxizitätsstufe entspricht (Ismerov N.F. u.a., 1977).

Die Ergebnisse der Untersuchung der chronischen Toxizität des Arzneimittels zeugen davon, daß dessen tägliche Anwendung innerhalb von 1 Monat in therapeutischer

und subtoxischer Desis keinen schädlichen Einfluß auf den funktionellen Zustand der Organe und Systeme des Organismus der Kaninchen und weißen Ratten ausgeübt hat. Das Niveau der untersuchten Indexe entsprach nach Beendigung der Versuche den Ausgangsangaben oder fiel mit ähnlichen Indexen in der Vergleichs- (Kontroll-)gruppe zusammen und überschritt nicht den von Zapdhjuk u.a. (1983) angeführten Bereich der physiologischen Normschwankungen bei diesen Tierarten. Das nachgewiesene höhere Niveau des direkt und indirekt reagierenden Blutserumbilirubins ($P > 0,05$) sowie eine etwas höhere Aktivität der Aminotransferasen ($P \quad 0,05$) in beiden Tiergruppen (Kaninchen und Ratten), die mit subtoxischen Arzneimitteldosen behandelt wurden, betrachten wir als eine bestimmte Tendenz zum negativen Einfluß einer langwährenden Einnahme subtoxischer Dosen auf die Leberfunktion. Die pathomorphologischen Untersuchungen der inneren Organe der Versuchs- und Kontrolltiere beider Arten wiesen keine deutlichen Veränderungen bei der Anwendung sowohl des Arzneimittels selbst als auch seiner Grundlage auf.

Die Untersuchungen haben festgestellt, daß das Arzneimittel beim Auftragen auf die Haut und beim Einführen in die Höhlen (in die Scheibe und ins Rektum) den Kaninchen und Ratten keine lokale Reizwirkung besitzt. Die morphologische Untersuchung der Hautgegenden, die bei den Versuchskaninchen und -ratten nach dem einmonatigen Auftragen des Arzneimittels auf diese Gegenden durchgeführt wurde, stellte keine Veränderungen fest. Es wurden auch keine Störungen der histologischen Struktur der Scheidenschleimhaut (die Einführungsstelle des Arzneimittels bei Weibchen) und der Rektumschleimhaut (die Einführungsstelle des Arzneimittels bei Männchen) nachgewiesen. Sowohl die einmalige als auch die zweimalige Einführung des Arzneimittels in therapeutischer Konzentration in den Konjunktivalraum beider Augen der Kaninchen und der Meerschweinchen wurde von einem kurzzeitigen (1 bis 2 min) Tränenfluß und einer unbedeutenden Rötung der Schleimhaut begleitet.

Bei der Einführung in den Konjunktivalraum der Kaninchen des Arzneimittels, in dem die Konzentration des Antiseptikums die therapeutischen Konzentration um das 10fahe übersteigt, wurde eine deutlicher ausgeprägte Hyperämie der Augenschleimhaut und eine längere Dauer dieser Reaktion (bis 1,5-3 Stunden) festgestellt. Die Untersuchung der Histostruktur der Augengewebe der Versuchstiere zeigte, daß die nachgewiesenen Veränderungen von einem funktionellen Charakter waren, sowohl bei der Anwendung des Arzneimittels in der therapeutischen Konzentration als auch in der Konzentration, die die letztere um das 10fache überstieg. Es waren keine Veränderungen vom destruktiven Charakter in den Komponenten der Schleimhaut zu verzeichnen.

Auf diese Weise legte die Untersuchung der lokalen Reizwirkung des Arzneimittels an drei Tierarten deren Ausbleibe sowohl bei der Einwirkung auf die Haut als auch auf die Schleimhäute der Augen, der Scheibe und des Rektums der Versuchstiere an den Tag.

Die Untersuchung des allergisierenden Einflusses des Arneimittels an 24 Meerschweinchen zeigte, daß seine Anwendung in therapeutischen Dosen von keinen Gesamtreaktionen der Tiere keinen Veränderungen seitens des Bluts begleitet wurden, die Zellentests im Reagenzglas blieben in den Versuchen ohne Veränderungen. Zugleich wurde festgestellt, daß die Arzneimitteldosen, die die therapeutische Dosis mindestens um das 10fache übersteigen, in bestimmten Maße die Entwicklung der Merkmale allergischer Äußerungen bedingen können, was durch die Erhöhung der Leukozytenagglomeration mindestens um das 4-fache, die Verminderung der Leukozytenzahl um 1,5 Tausend in 1 $mm^3$ Blut, durch den Anstieg der Thrombozytenzahl bis 70 %, die Erhöhung des Eosinophilengehalts mindestens um das 4-fache ($P < 0{,}05$) gekennzeichnet ist.

Das Arzneimittel in therapeutischer und selbst in subtoxischer Dosis übt keinen Einfluß auf den Mechanismus der Immunität aus: es wurden keine Veränderungen sowohl der Zahl der immunokompetenten Zellen als auch deren Funktion, insbesondere der Synthese der Hauptklassen der Antikörper beobachtet. Es wurden auch keine Merkmale der Hemmung der

unspezifischen Schutzfaktoren im Organismus der Tiere festgestellt. Die Ergebnisse der histologischen Untersuchungen der Milz und der Lymphknoten, in welchen keine makrophagale und lymphoide Infiltration und Plasmazellenreaktion zu verzeichnen waren, bestätigen in bestimmtem Maße die Ergebnisse der immunologischen Tests.

Das Arzneimittel wird beim Auftragen sowohl auf die Haut als auch auf die Schleimhaut der Kaninchen resorbiert. Jedoch geht die Resorption durch die Haut langsamer und in geringeren Mengen als durch die Schleimhaut vor sich. Es wurde auch festestellt, daß obwohl die Resorption des Arzneimittels durch die Schleimhaut verhältnismäßig schneller als durch die Haut vonstatten geht, läuft der Resorptionsprozeß doch langsam ab.

Die experimentellen Untersuchungen bestätigten eine hohe spezifische Wirksamkeit des erfindungsgemäßen Arzneimittels in bezug auf die Erreger von Syphilis, Gonorrhoe und Trichomoniasis.

Die Untersuchung des schädlichen Einflusses des Arzneimittels auf den Organismus der Labortiere zeigte, daß dieses in Übereinstimmung mit den Ergebnissen der Untersuchung der akuten Toxizität als verhältnismäßig unschädlich eingeschätzt werden kann, und seine langwierige Anwendung in therapeutischen und sogar subtoxischen Dosen von keinem ausgeprägten schädlichen Einfluß auf die Organe und Systeme der Versuchstiere begleitet wird.

Die Ergebnisse der vorklinischen Untersuchungen des Arzneimittels zeigten, daß am effektivsten für die Prophylaxe venerischer Erkrankungen das Arzneimittel in flüssiger Arzneiform folgender Zusammensetzung (in Masse%) vorgeschlagen werden kann:

para-Nitro- -chlorzimtaldehyd der Formel 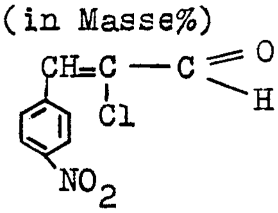 0,3

Dimethylsulfoxid 5,0

Polyäthylenglykol von 400 Molekularmasse bis 100,0

Zur Prophylaxe venerischer Erkrankungen und Behandlung der urogenitalen Trichomoniasis bei Frauen wird das Arzneimittel in Aerosolverpackung unter Druck in flüssiger Form folgender Zusammensetzung (in Masse%) empfohlen:

para-Nitro-α-chlorzimtaldehyd der Formel

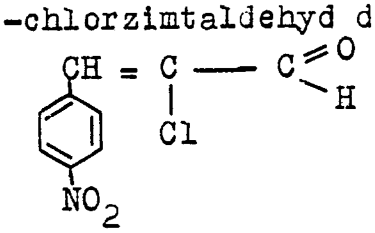

0,3

| | |
|---|---|
| Dimethylsulfoxid | 5,0 |
| Polyäthylenglykol von 400 Molekularmasse | 10,0-30,0 |
| oberflächenaktive Substanz | 3,0-6,0 |

Wasser bis 100,0 und Propellent in einer von 3 bis 10 % Menge von der Gesamtmasse der Komponenten.

Klinische Prüfungen des Arzneimittels als Mittel zur individuellen Prophylaxe venerischer Erkrankungen wurden in großen dermatovenerologischen Anstalten der Sowjetunion durchgeführt. Die Untersuchung wurde in Stationen der individuellen Prophylaxe venerischer Erkrankungen durchgeführt, wo den Besuchern die prophylaktische Hilfe vom Personal dieser Stationen geleistet wurde sowie das Arzneimittel den Personen gegeben wurde, die unordentliche Geschlechtsverbindungen eingehen. Die Untersuchungen zeigten, daß unter den unter Beobachtung stehenden Männern, die zufällige Geschlechtsverbindungen hatten und das Arzneimittel anwendeten, keine Fälle der Infizierung an Syphilis, Honorrhoe und Trichomoniasis nachgewiesen wurden. Es wurden auch keine Merkmale der toxischen, allergisierenden und lokalen Reizwirkung des Arzneimittels auf den Organismus der Patienten festgestellt.

Die Ergebnisse der Behandlung der urogenitalen Trichomoniasis bei 105 Patienten nach dem Schema mit Anwendung des erfindungsgemaßen Arzneimittels in einer Menge von 1,5 bis 10 ml zweimal täglich im Laufe von 5 bis 7 Tagen zeigten eine hohe therapeutische protistozide Effektivität, die in einer vollen ätiologischen und klinischen Ausheilung der Kranken bestand. Es wurden keine Fälle der Rezidivierung der Trichomoniasis bei einer ang dauernden (3 Monaten) Kontrollbeobachtung der Kranken sowie keine Nebenerscheinungen verzeichnet.

## Industrielle Anwendbarkeit

Das erfindungsgemaße Arzneimittel wird in der dermatovenerologischen und gynäkologischen Praxis Verwendung finden.

PATENTANSPRÜCHE

1. Arzneimittel zur individuellen Prophylaxe venerischer Erkrankungen und zur Behandlung der urogenitalen Trichomoniasis, enthaltend eine aktive Komponente und ein pharmazeutisches Verdünnungsmittel, dadurch gekennzeichnet, daß es eine effektive Menge der aktiven Komponente enthält, welche ein synergisches Gemisch aus para-Nitro-α-chlorzimtaldehyd der Formel

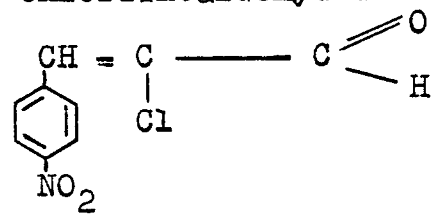

und Dimethylsulfoxid darstellt.

2. Arzneimittel nach Anspruch 1, dadurch gekennzeichnet, daß es Polyäthylenglykol mit der Molekularmasse 400 als pharmazeutisches Verdünnungsmittel enthält.

3. Arzneimittel nach Anspruch 1, dadurch gekennzeichnet, daß es Komponenten bei folgendem Verhältnis (Masse-%) enthält:

| | |
|---|---|
| para-Nitro-α-chlorzimtaldehyd | 0,3 |
| Dimethylsulfoxid | 5,0 |
| Polyäthylenglykol mit der Molekularmasse 400 | bis 100. |

4. Arzneimittel nach Ansprüchen 1,2 in der Aerosolverpackung unter Druck in flüssiger Form, dadurch gekennzeichnet, daß es eine oberflächenaktive Substanz und Wasser bei folgendem Komponentenverhältnis /Masse-%) zusätzlich enthält:

| | |
|---|---|
| para-Nitro-α-chlorzimtaldehyd | 0,3 |
| Dimethylsulfoxid | 5,0 |
| Polyäthylen mit der Molekularmasse 400 | 10-30 |
| oberflächenaktive Substanz | 3,0-6,0 |
| Wasser | bis 100, |

sowie Propellent in einer Menge von 3 bis 10 % von der Gesamtmasse der Komponenten.

# INTERNATIONAL SEARCH REPORT

International Application No PCT/SU 87/00151

**I. CLASSIFICATION OF SUBJECT MATTER** (if several classification symbols apply, indicate all) [6]

According to International Patent Classification (IPC) or to both National Classification and IPC

$IPC^4$ A 61 K 47/00, 9/12 // (A 61 K 47/00, 31:11, 31:10)

**II. FIELDS SEARCHED**

Minimum Documentation Searched [7]

| Classification System | Classification Symbols |
|---|---|
| $IPC^4$ | A 61 K 47/00, 9/12, 31/10, 31/11, 31/47 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | US, A, 4575515, (Clark Pharmaceutical Laboratories Ltd.), 11 March 1986 (11.03.86), see the claims | 1-3 |
| A | SU, A1, 102259, (G.N. Pershin et al), 4 August 1951 (04.08.51), see the claims | 1 |
| A | SU, A1, 110747, (G.N. Pershin et al), 28 April 1956 (28.04.56), see the claims | 1 |
| A | EP, A3, 0134964, (BASF WYANDOTTE CORPORATION), 27 March 1985 (27.03.85), see the claims | 4 |
| A | US, A, 4581225, (Eli Lilly and Company), 8 April 1986 (08.04.86), see the claims | 4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

**IV. CERTIFICATION**

| Date of the Actual Completion of the International Search | Date of Mailing of this International Search Report |
|---|---|
| 22 July 1988 (22.07.88) | 1 September 1988 (01.09.88) |
| **International Searching Authority** | **Signature of Authorized Officer** |
| ISA/SU | |